(19) Europäisches Patentamt
European Patent Office
Office européen des brevets

(11) Veröffentlichungsnummer: **0 374 890 A1**

(12) **EUROPÄISCHE PATENTANMELDUNG**

(21) Anmeldenummer: **89123564.0**

(22) Anmeldetag: **20.12.89**

(51) Int. Cl.5: **C07C 46/10, C07C 50/34, A61K 31/12**

(30) Priorität: **21.12.88 GB 8829782**

(43) Veröffentlichungstag der Anmeldung: **27.06.90 Patentblatt 90/26**

(84) Benannte Vertragsstaaten: **AT BE CH DE ES FR GB GR IT LI LU NL SE**

(71) Anmelder: **GERUM ESTABLISHMENT**
**Landstrasse 153**
**FL-9491 Ruggell(LI)**

(72) Erfinder: **Alexa, M., Dr.**
**Frombachstrasse 64**
**D-7746 Hornberg(DE)**

(74) Vertreter: **Büchel, Kurt F., Dr.**
**Bergstrasse 297**
**FL-9495 Triesen(LI)**

(54) **Verfahren zur Herstellung von Anthrachinon-Derivaten.**

(57) Anthrachinonderivate der Formel

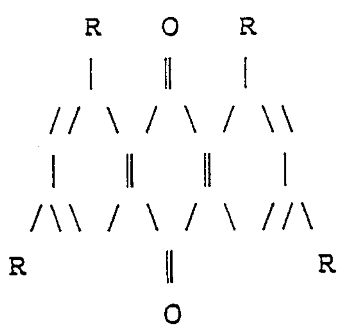

wobei höchstens zwei R aus der Gruppe von H, CH3, C2H5 und C3H7, jedoch mindestens zwei R aus der Gruppe der Substituenden OH, CH2OH, C2H4OH, den Estern dieser Substituenden mit Carbonsäuren von maximal 3 C-Atomen oder den Glykosiden dieser Substituenden stammen, wobei eine das Anthrachinonderivat enthaltende Pflanze oder ein Extrakt dieser Pflanze mittels eines Lösungsmittels - insbesondere bei erhöhter Temperatur - extrahiert wird, werden nach folgendem Verfahren hergestellt:

a) es wird mittels eines ersten, hydrophilen Lösungsmittels, insbesondere mittels Wasser, einem Alkohol mit maximal 3 C-Atomen, Aceton, Äthylazetat, Diäthylazetat, bzw. Mischungen dieser Lösungsmittel extrahiert und anschliessend filtriert;

b) die resultierende Lösung wird - vorzugsweise bis zu einem Restgehalt von mindestens 5, insbesondere auf 10 bis 15 Gewichtsprozent Lösungsmittel zu einer sirupartigen Konsistenz - entweder eingedampft oder über einen polaren Ionenaustauscher, z.B. Amberlite IR45, geleitet, aus dem die zurückgehaltenen Anthrachinonderivate mit Methanol eluiert werden;

c) aus dem Sirup wird die Verbindung mittels eines zweiten Lösungsmittels, insbesondere Benzol und/oder eines Homologen davon, extrahiert, welcher Schritt vorzugsweise mit dem Rückstand der Extraktion 2 bis 4 mal wiederholt wird;

d) der Extrakt wird zur Trockne eingedampft und gegebenenfalls umkristallisiert.

Das sich nach dem Schritt a) ergebende Filtrat kann vor dem Schritt b) - insbesondere mehrmals nacheinander - mehrere Tage lang Luft und Licht ausgesetzt werden, vorzugsweise unter Rühren und/oder Sauerstoffeintrag und/oder UV-Bestrahlung.

EP 0 374 890 A1

# Verfahren zur Herstellung von Anthrachinon-Derivaten

Die Erfindung betrifft ein Verfahren zur Herstellung von Anthrachinon-Derivaten aus entsprechenden Extrakten oder Pflanzenteilen nach dem Oberbegriff des Anspruches 1. Insbesondere, aber nicht ausschliesslich, betrifft die Erfindung die Isolierung von Emodinen, wie z.B. von Aloe-Emodin, Frangula-Emodin oder Chrysophanol, oder von Aloin Diastereomer A, Aloeresin A und B, sowie deren Verwendung für die Herstellung von Arzneimitteln.

Aloe-Emodin wurde bisher durch Extraktion aus der Aloewurzel durch längeres Kochen in einer Eisen(III)-Chloridlösung gewonnen (Wolfgang Widmayer, "Zur Kenntnis der Analytik von Aloin", Dissertation an der Universität Tübingen, 1975, Seite 67). Dies gelang mit einer Ausbeute von nur 12 bis 16% der Theorie; andere Inhaltsstoffe, wie insbesondere Aloeresin A und B, werden dabei zerstört.

In der DE-A-3316463 wurde die Verwendung einschlägiger Verbindungen zur Behandlung von Psoriasis und anderen Hautkrankheiten, in der DE-A-3447953 zur Behandlung von Schmerzen bei Verbrennungen, sowie gegen grampositive Bakterien und grampositive sporenbildende Stäbchen beschrieben.

Es besteht daher ein Interesse daran, die Ausbeute des genannten Herstellungsverfahrens zu verbessern und andere Inhaltsstoffe, wie beispielsweise Aloeresin A und B, die z.B. für die Behandlung von Ulcus duodeni eingesetzt werden können, nicht zu zerstören. Dies gelingt durch die im Kennzeichen des Anspruches 1 beschriebenen Massnahmen, die die Anthrachinonderivate löslich machen. Vorteilhafte Weiterbildungen der Erfindung sind in den Unteransprüchen beschrieben.

Die Erfindung wird im folgenden anhand von Beispielen näher erläutert:

## Beispiel 1

1 kg Aloeextrakt (z.B. Curacao-Aloe oder Kap-Aloe) wird mit 10 Liter Methanol am Rückflusskühler während 2 Stunden gekocht. Man filtriert und behandelt den Rückstand ein zweites Mal in gleicher Weise. Die Lösungen werden vereinigt, filtriert, bis zu 4 Wochen dem Sonnenlicht ausgesetzt und dann unter vermindertem Druck bis zu einer sirupartigen Konsistenz (etwa 10% Methanol enthaltend) eingeengt. Engt man zuwenig ein, dann mischt sich die Methanolphase mit der anschliessenden Toluolphase, was natürlich unerwünscht ist. Engt man zuviel ein, ist die Löslichkeit des Rückstandes in Toluol unerwünscht reduziert. Der Sirup wird mehrmals bei Raumtemperatur im Schütteltrichter mit 500 ml Toluol extrahiert, und zwar so lange, bis die Toluolphase keine Färbung mehr zeigt. Die Toluol-Fraktionen werden gesammelt und unter vermindertem Druck zur Trockene eingedampft. Der Rückstand enthält fast reines Aloe-Emodin. Er wird in 100 ml Methanol bei 50 °C gelöst und durch Abkühlen auskristallisiert. Man filtriert und kristallisiert gewünschtenfalls noch einmal um. Es ergeben sich 5,36 g (das sind etwa 50% der theoretischen Ausbeute) reines Aloe-Emodin.

## Beispiel 2

Dasselbe Vorgehen nach Beispiel 1 mit Isopropylalkohol statt Methanol ergibt 4.88 g Aloe-Emodin.

## Beispiel 3

1 Kg Aloeextrakt wird in 5 Liter kochendem Wasser gelöst. Das Filtrat wird vor der Behandlung mit Sonnenlicht über 500 ml einer unpolaren Ionenaustauscher-Schüttung (z.B. Amberlite XAD7) laufen gelassen, wobei Aloeresin B in der Lösung bleibt und nicht adsorbiert wird; es kann aus der Lösung durch Eindampfen und Umkristallisieren rein gewonnen werden. Der Ionenaustauscher wird mit Methanol wieder rein gewaschen und die Lösung nach Beispiel 1 weiterbehandelt. Beim Extrahieren des eingedickten Methanol-Sirups mit Toluol (Schritt c) bleibt Aloeresin A in der Methanolphase und kann in analoger Weise anschliessend rein gewonnen werden.

## Beispiel 4

Der Rückstand der Toluolextraktion aus Beispiel 1 oder 3 wird neuerlich in Methanol aufgenommen und weitere 2 bis 4 Wochen lang Luft und Licht ausgesetzt. Die Weiterbehandlung erfolgt dann wie in Beispiel 1 oder 3 erwähnt. Nach 2 bis 3-maligem Behandeln ist praktisch 100% des ursprünglich vorgelegenen Aloins in Aloe-Emodin umgewandelt.

## Beispiel 5

1 kg Aloeextrakt wird in 5 Liter Wasser 1 Stunde lang am Rückflusskühler gekocht; die Lösung wird filtriert, anschliessend mit 500 ml Toluol

oder Benzol versetzt und mehrere 100 Stunden unter Rühren und bei Raumtemperatur einer UV-Bestrahlung ausgesetzt. Das entstehende Aloe-Emodin geht in die Toluolphase, die sich dabei zunächst gelb, später rot bis purpurrot färbt. Die Toluolphase wird abgetrennt und zur Trockne gedampft; man wiederholt den Vorgang nach jeweils 100 bis 200 Stunden mit 500 ml reinem Toluol oder Benzol; es kann das Destillat des Eindampfschrittes wiederverwendet werden, und zwar so lange, bis keine Verfärbung mehr eintritt, meist nach zwei bis drei Zyklen.

Der Rückstand aus der Toluolphase wird in 100 ml Methanol bei 50 °C aufgenommen; beim Erkalten fällt reines Aloe-Emodin aus; es kann gewünschtenfalls noch mit Methanol gewaschen und/oder umkristallisiert werden. Die dunkelroten Kristalle haben ein Molekulargewicht von 272 und einen Schmelzpunkt von 236 °C.

Beispiel 6

In analoger Weise können aus Frangula-Extrakt (Faulbaumrinde) Frangula-Emodin, Frangulin A und B bzw. Gluco-Frangulin A und B gewonnen werden.

Beispiel 7

1 Kg Aloeextrakt wird mit 10 Liter Methanol heiss extrahiert und filtriert. Das Filtrat wird unter vermindertem Druck bis zu einer sirupartigen Konsistenz eingeengt, mit wenig Chloroform vermischt (ca. 10 ml) und bei -18 °C bis 22 °C 24 Stunden lang abgekühlt. Es kristallisiert Aloin Diastereomer A. Es ergeben sich 89 g Aloin Diastereomer A, das sind fast 100% der theoretischen Ausbeute.

Ein mit 5 bis 10 mg dieser Wirksubstanz pro Tablette oder Gelatinekapsel hergestelltes Arzneimittel wirkt bei einmaliger Dos is bei freiwilligen Probanden je nach deren spezieller Sensibilisierng nach 2 bis 24 Stunden. Die eingedickten Faeces werden aufgeweicht, die Defäkation wird in Gang gesetzt.

Beispiel 8

Bei gleichem Vorgehen wie nach Beispiel 1, wenn jedoch dem Methanol 10 bis 20, höchstens 25 % eines chlorierten Lösungsmittels, insbesondere Chloroform oder Dichlormethan zugesetzt werden, ist die Löslichkeit für andere Substanzen weiter herabgesetzt, und die Methanolphase enthält neben den Aloe-Bestandteilen weniger Verunreinigungen. Verwendet man Äthanol, dann dürfen maximal 10 Gewichtsprozent eines chlorierten Lösungsmittels zugefügt werden, bei Verwendung von Äthylazetat überhaupt kein chloriertes Lösungsmittel, da Äthylazetat selbst schon nur eine beschränkte Lösekraft für Aloe-Emodin besitzt.

Beispiel 9

Je 100 g einer handelsüblichen Salbengrundlage werden alternativ mit 50 mg Aloin Diastereomer A; 50 mg Aloe-Emodin; 50 mg Aloin A und 25 mg Aloe Emodin; beziehungsweise mit 50 mg Aloin A und 0.2 % Parabene versetzt. Nach 3 Jahren ist das Produkt mit Aloin A von Aspergilius Niger kontaminiert, während die anderen drei Mischungen keine Kontamination aufweisen. Aloe- Emodin ist also als Konservierungsmittel um eine Zehnerpotenz wirksamer als das Parabene.

Beispiel 10

Die Konzentration des Wirkstoffes Aloe-Emodin in einer Salbengrundlage kann von 0,025 bis 2% betragen. Die therapeutische Anwendung erfolgt bei Neurodermitis in der Weise, dass die erkrankten Hautpartien 1 bis 2 mal pro Tag mit dem Arzneimittel bestrichen werden, wobei die von Neurodermitis befallenen Hautpartien abends mit dem Arzneimittel bestrichen werden und über Nacht abgedeckt einwirken sollen. Die befallenen Hautpartien werden kurz nach der Behandlung dunkelrot und schmerzen teilweise. Nach ca. 3 bis 5 Tagen Behandlungsdauer findet eine Regeneration und Epitelisierung statt. Gesunde Hautpartien werden von dem Arzneimittel der Erfindung nicht berührt. Kein Konsverierungsmittel ist erforderlich.

## Ansprüche

1. Verfahren zur Herstellung von Anthrachinonderivaten der allgemeinen Formel

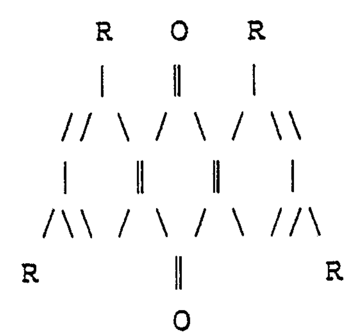

wobei höchstens zwei R aus der Gruppe von H,

CH3, C2H5 und C3H7, jedoch mindestens zwei R aus der Gruppe der Substituenden OH, CH2OH, C2H4OH, den Estern dieser Substituenden mit Carbonsäuren von maximal 3 C-Atomen oder den Glykosiden dieser Substituenden stammen, wobei eine das Anthrachinonderivat enthaltende Pflanze oder ein Extrakt dieser Pflanze mittels eines Lösungsmittels - insbesondere bei erhöhter Temperatur -extrahiert wird, gekennzeichnet durch die Kombination der folgenden Schritte:

a) es wird mittels eines ersten, hydrophilen Lösungsmittels, insbesondere mittels Wasser, einem Alkohol mit maximal 3 C-Atomen, Aceton, Äthylazetat, Diäthylazetat, bzw. Mischungen dieser Lösungsmittel extrahiert und anschliessend filtriert;

b) die resultierende Lösung wird - vorzugsweise bis zu einem Restgehalt von mindestens 5, insbesondere auf 10 bis 15 Gewichtsprozent Lösungsmittel zu einer sirupartigen Konsistenz - entweder eingedampft oder über einen polaren Ionenaustauscher, z.B. Amberlite IR45, geleitet, aus dem die zurückgehaltenen Anthrachinonderivate mit Methanol eluiert werden;

c) aus dem Sirup wird die Verbindung mittels eines zweiten Lösungsmittels, insbesondere Benzol und/oder eines Homologen davon, extrahiert, welcher Schritt vorzugsweise mit dem Rückstand der Extraktion zwei- bis viermal wiederholt wird;

d) der Extrakt wird zur Trockne eingedampft und gegebenenfalls umkristallisiert.

2. Verfahren nach Anspruch 1, dadurch gekennzeichnet, dass im Schritt a) ein Gewichtsteil der Pflanze bzw. des Pflanzenextraktes mit 5 bis 10 Gewichtsteilen des ersten Lösungsmittels extrahiert wird, dem gegebenenfalls 5 bis 50, vorzugsweise maximal 10 bis 25 Gewichtsprozent eines chlorierten Lösungsmittels, insbesondere Chloroform oder Dichlormethan, zugefügt werden, und dass der Schritt a) mit dem Rückstand der Filtration vorzugsweise zwei- bis viermal wiederholt wird.

3. Verfahren nach Anspruch 1 oder 2, dadurch gekennzeichnet, dass das sich nach dem Schritt a) ergebende Filtrat vor Durchführung des Schrittes b) wenigstens einen, vorzugsweise 5 bis 10, insbesondere 20 bis 30 Tage lang Luft und Licht ausgesetzt wird, vorzugsweise unter Rühren und/oder Sauerstoffeintrag und/oder UV-Bestrahlung.

4. Verfahren nach einem der vorhergehenden Ansprüche, dadurch gekennzeichnet, dass die sich aus Schritt a) ergebende Lösung über einen unpolaren Ionenaustauscher, insbesondere über Amberlite XAD7, geleitet, aus der verbleibenden Lösung durch Eindampfen und gegebenenfalls Umkristallisieren Aloeresin B gewonnen wird, während die auf dem Ionenaustauscher zurückgehaltenen Verbindungen mit Methanol ausgewaschen und den Schritten b) bis d) unterworfen werden, wobei gegebenenfalls Aloeresin A nach dem Schritt c) aus der Phase des ersten Lösungsmittels rein gewonnen wird (Beispiel 3).

5. Verfahren nach Anspruch 1 oder 2, dadurch gekennzeichnet, dass der sich aus dem Schritt b) ergebende Sirup bis zu einer beginnenden Trübung mit einem chlorierten Lösungsmittel, insbesondere mit Chloroform oder Dichlormethan, versetzt und anschliessend auf eine Temperatur unter dem Gefrierpunkt, insbesondere auf -15 bis -25° C abgekühlt wird, wobei Aloin-Diastereomer A auskristallisiert und abfiltriert, sowie gegebenenfalls umkristallisiert wird, während gegebenenfalls das in der überstehenden Lösung verbleibende Aloin Diastereomer B bei Raum- oder erhöhter Temperatur zu jeweils 50% in Aloin Diastereomer A umgelagert und dieses durch Wiederholung des Abkühlens und Auskristallisierens abgetrennt wird. (Beispiel 7)

6. Verwendung von Aloeresin A und/oder Aloeresin B für die Herstellung von Arzneimitteln zur Behandlung von Ulcus duodeni.

7. Verwendung von Aloin-Diastereomer A der Formel

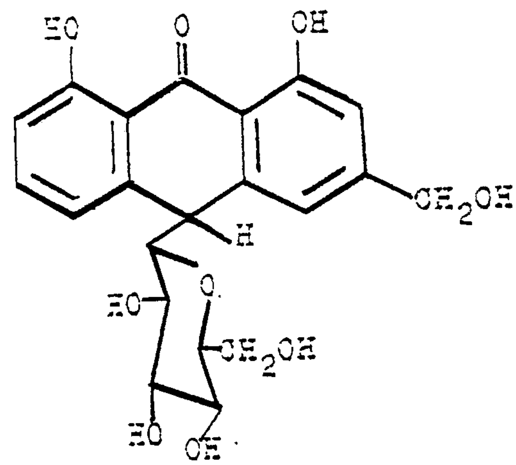

für die Herstellung von Arzneimitteln zur Behandlung akuter und chronischer Obstipation, Hämorrhoiden, Analfissuren, Hernien, Hypertension, Gefäss-Sklerose und Postpartum.

8. Verwendung von Aloe-Emodin in einer Konzentration von 0.05 Gewichtsprozent als Konservierungsmittel.

Europäisches Patentamt

# EUROPÄISCHER RECHERCHENBERICHT

Nummer der Anmeldung

EP 89123564.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| D,A | DE - A1 - 3 447 953<br>(ALEXA,MIHAI)<br>* Zusammenfassung *<br>-- | 1,6 | C 07 C 46/10<br>C 07 C 50/34<br>A 61 K 31/12 |
| A | DE - A1 - 3 315 463<br>(ALEXA,MIHAI)<br>* Zusammenfassung *<br>-- | 1,6 | |
| A | CHEMICAL ABSTRACTS, Band 90, Nr. 8, 19. Februar 1979, Columbus, Ohio, USA<br>R.S.SCHREIBER "Oral compositions containing on anticalculus agent."<br>Seite 392, Zusammenfassung Nr. 61 242k<br>& US-A-4 105 758<br>-- | 1,6 | |
| A | CHEMICAL ABSTRACTS, Band 104, Nr. 23, 9. Juni 1986, Columbus, Ohio, USA<br>D.LORENZ et al. "Pharamco-kinetic studies of alizarin in man."<br>Seiten 14,15, Zusammenfassung Nr. 199 515r<br>& Methods Find. Exp. Clin. Pharmacol. 1985,m 7(12), 637-43<br>-- | 1,6 | **RECHERCHIERTE SACHGEBIETE (Int Cl 5)**<br>C 07 C 46/00<br>C 07 C 50/00<br>A 61 K |
| A | CHEMICAL ABSTRACTS, Band 101, Nr. 17, 22. Oktober 1984, Columbus, Ohio, USA<br>M.KUBO et al. "Studies on scutellariae radix. VII.Anti-arthritic and anti-inflamma-tory actions of methanolic extract and flavonoid components from Scutellariae radix." | 1,2,6 | |

Der vorliegende Recherchenbericht wurde für alle Patentansprüche erstellt.

| Recherchenort | Abschlußdatum der Recherche | Prüfer |
|---|---|---|
| VIENNA | 28-03-1990 | REIF |

KATEGORIE DER GENANNTEN DOKUMENTEN

- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

-2-
EP 89123564.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| | Seite 42, Zusammenfassung Nr. 143 768w & Chem. Pharm. Bull. 1984, 32(7), 2724-9 ---- | | |
| | | | RECHERCHIERTE SACHGEBIETE (Int Cl 5) |

Der vorliegende Recherchenbericht wurde für alle Patentansprüche erstellt.

| Recherchenort | Abschlußdatum der Recherche | Prüfer |
|---|---|---|
| VIENNA | 28-03-1990 | REIF |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82